## Europäisches Patentamt

## European Patent Office

## Office européen des brevets

(19)

(11) Veröffentlichungsnummer: **0 048 804**
**B1**

(12) **EUROPÄISCHE PATENTSCHRIFT**

(45) Veröffentlichungstag der Patentschrift:
**14.12.83**

(51) Int. Cl.³: **C 07 C 35/20,** C 07 C 29/04

(21) Anmeldenummer: **81106051.6**

(22) Anmeldetag: **01.08.81**

(54) **Verfahren zur Herstellung von Cycloocten-4-ol-1 aus Cyclooctadien-1,5.**

(30) Priorität: **01.10.80 DE 3037093**

(43) Veröffentlichungstag der Anmeldung:
**07.04.82 Patentblatt 82/14**

(45) Bekanntmachung des Hinweises auf die Patenterteilung:
**14.12.83 Patentblatt 83/50**

(84) Benannte Vertragsstaaten:
**BE DE FR GB IT NL**

(56) Entgegenhaltungen:
**DE - B - 1 643 617**
**US - A - 3 301 887**

(73) Patentinhaber: **CHEMISCHE WERKE HÜLS AG,**
**Postfach 1320, D-4370 Marl 1 (DE)**

(72) Erfinder: **Kaufhold, Manfred, Dr., Bitterfelder Strasse 7a,**
**D-4370 Marl (DE)**

## Verfahren zur Herstellung von Cycloocten-4-ol-1 aus Cyclooctadien-1,5

Es ist bekannt, zur Herstellung von Cycloocten-4-ol-1 in einer ersten Stufe zunächst in Gegenwart von Perchlorsäure 1 Mol Ameisensäure an 1 Mol cis-cis-1,5-Cyclooctadien zu addieren. Danach werden die Reaktionsprodukte verseift und durch sorgfältige fraktionierte Destillation aufgearbeitet. Zur Trennung der in grosser Menge gebildeten gesättigten Alkohole von dem gewünschten Cycloocten-4-ol-1 ist eine aufwendige Extraktion mit einer 20prozentigen wässrigen Silbernitratlösung erforderlich. Die Ausbeute an Cycloocten-4-ol-1 beträgt bei diesem aufwendigen Verfahren nur 20% (A.C. Cope und P.E. Peterson, Journal of the American Chemical Society 81 (1959), Seite 1 643 bis 1 650).

Auch die unkatalysierte Addition von Ameisensäure an andere Olefine, jedoch nicht an Cyclooctadien-1,5, ist bekannt (H.B. Knight, R.E. Koos und D. Swern, Journal of the American Chemical Society 75 (1953), Seite 6 212). Bei der unkatalysierten Reaktion erreicht man beim Siedepunkt der Ameisensäure erst nach 24 Stunden einen Umsatz von 80%. Durch Zusatz von kleinen Mengen Perchlorsäure (0,5 bis 2 Gewichtsprozent, bezogen auf das Olefin) kann man die Reaktionszeit auf 5 bis 15 Minuten reduzieren.

Bei einem Isomeren des Cyclooctadiens-1,5, dem Vinylcyclohexen erhält man nach der US-PS 2 764 610 durch Erhitzen von 1 Mol Vinylcyclohexen und 3 Mol Ameisensäure (8 Stunden bei 94 bis 97 °C unter Rückfluss) einen Umsatz an Vinylcyclohexen von 44%. Die Ausbeute an Monoameisensäureester, bezogen auf umgesetztes Vinylcyclohexen beträgt nur 61%.

Alle bekannten Verfahren ergeben somit nur geringe Ausbeuten, benötigen lange Reaktionszeiten oder verwenden Katalysatoren, die Aufarbeitungs- und Umweltprobleme mit sich bringen.

Es besteht daher ein grosses Interesse an einem Verfahren, nach dem man bei geringem technischen Aufwand ohne lange Verweilzeiten ein Cycloocten-4-ol-1 mit einem Gehalt über 70% in hoher Ausbeute herstellen kann.

Die sich hieraus ergebende Aufgabe wird erfindungsgemäss entsprechend den Angaben der Patentansprüche gelöst.

Überraschenderweise erhält man Cycloocten-4-ol-1 mit einem durch Kernresonanz-Spektroskopie ermittelten Gehalt von über 70% in einfacher Weise und mit guten Ausbeuten, indem man Cyclooctadien-1,5 ohne Katalysatoren bei Temperaturen von 50 bis 200 °C, vorzugsweise von 90 bis 105 °C, mit Ameisensäure umsetzt, ohne weitere Aufarbeitung den Ameisensäureester direkt durch Destillation gewinnt und in üblicher Weise mit einem niedrig siedenden Alkohol zu dessen Formiat und zum Cycloocten-4-ol-1 umestert.

Dieser Befund ist neu und überraschend. Bisher wurde immer gelehrt, dass die Addition von Ameisensäure an z.B. Cyclooctadien-1,5 unabhängig von den Reaktionsbedingungen wie Temperatur, Konzentrationen und Mineralsäurezusatz stets über ein bestimmtes, stabiles Carboniumion als intermediäre produktbestimmende Verbindung verläuft und deshalb eine Veränderung der Reaktionsbedingungen nicht zu einer Verschiebung des Produktspektrums führen kann. H.W. Gibson erklärt in einer Arbeit über die Chemie der Ameisensäure und ihrer einfachen Derivate (Chemical Rev. 69 (1969), Seite 673 bis 683), dass bei der Addition von Ameisensäure an ein Molekül mit zwei oder mehreren Doppelbindungen die Addition so erfolgt, dass das stabilste Carboniumion als intermediäre Verbindung auftritt. Dieses Carboniumion ist im Falle der Reaktion von Cyclooctadien-1,5 in der ersten o.g. Literaturstelle beschrieben. Aus dem zuerst entstehenden Cyclooctenylkation bildet sich nach Meinung der Fachleute das stabilere Bicyclooctylkation. Auf diese Weise wird erklärt, warum bei der sauer katalysierten Addition von Ameisensäure an Cyclooctadien-1,5 Bicyclooctylformiat entsteht.

Neben den bicyclischen Verbindungen entstehen nach Angaben der o.g. Autoren noch eine ganze Reihe anderer Nebenprodukte sowie hochsiedende und nicht destillier- und nicht identifizierbare harzartige Produkte. Daher war es überraschend, dass bei dem erfindungsgemässen Verfahren trotz gleicher Zwischenstufen und vergleichbarem Reaktionsablauf höhere Ausbeuten erzielt werden.

Während Cope und Peterson bei kurzen nicht genau angegebenen Reaktionszeiten in Gegenwart von Katalysatoren (s.o.) Ausbeuten von 20% erreichen, werden bei dem erfindungsgemässen Verfahren ohne Katalysator Ausbeuten bezogen auf umgesetztes Cyclooctadien von über 75% erzielt, wobei als Reaktionszeit für die Umsetzung von Dien mit Ameisensäure im allgemeinen 10 Stunden ausreichen. Dieses Ergebnis steht im Gegensatz zu den Erfahrungen der Fachleute. Denn üblicherweise ist es so, dass bei Reaktionen, die zu Produkten führen, die weiterreagieren können, in diesem Fall das Cyclooctenylformiat, die Selektivität umso geringer wird, je länger die Verweilzeit ist.

Gegenüber der Addition ohne Katalysatoren von Ameisensäure an andere Olefine, jedoch nicht an Cyclooctadien-1,5 (Knight, Koos und Swern), ist die Reaktionszeit beim erfindungsgemässen Verfahren überraschenderweise weniger als halb so lang.

Das Weglassen des Katalysators bewirkt also überraschend positive Effekte und hat darüber hinaus den entscheidenden Vorteil, dass das Reaktionsprodukt sehr leicht – nämlich durch direkte Destillation – aufgearbeitet werden kann.

Deshalb ist der Chemikalienverbrauch niedrig, es entstehen keine Materialverluste, und es gibt keine Umweltprobleme infolge der Beseitigung des Katalysators.

Die Addition von Ameisensäure an Cyclooctadien erfolgt bei Temperaturen von 50 bis 200 °C, vorzugsweise bei 95 bis 105 °C.

Das erfindungsgemässe Verfahren führt man aus wirtschaftlichen Gründen bevorzugt bei Normaldruck durch, wobei die maximale Temperatur dann die Temperatur ist, bei der das Reaktionsgemisch siedet, d.h., in einem Bereich von 100 bis 105 °C. Temperaturen unter 50 °C sind nachteilig, weil sie die Reaktionszeit zu sehr verlängern. Bei Temperaturen über 105 °C arbeitet man unter Druck.

Das Molverhältnis Cyclooctadien zu Ameisensäure beträgt vorzugsweise 1:0,1 bis 1:10, insbesondere 1:1,5 bis 1:6. Es hat einen überraschend grossen Einfluss auf den Umsatz (s. Beispiele).

Man erhält das Cycloocten-4-ol-1 mit einem durch Kernresonanz-Spektroskopie ermittelten Gehalt von über 70%, von 71 bis 80%.

Das nach dem erfindungsgemässen Verfahren erhaltene Cycloocten-4-ol-1 ist ein wertvolles Zwischenprodukt für zahlreiche weitere technische Synthesen.

Beispiel 1

Man benutzt eine Glasapparatur, die aus einem Dreihalskolben mit Rührer, Thermometer und Rückflusskühler besteht.

Man setzt 1 296 g (12 Mol) Cyclooctadien-1,5 (99,76-prozentig) und 1 656 g (36 Mol) Ameisensäure (99,0-prozentig) ein.

Dieses Gemisch erhitzt man 10 Stunden lang unter Rühren und Stickstoffabdeckung am Rückfluss zum Sieden, wobei sich im Sumpf eine Temperatur von 102 °C einstellt. Das Gemisch ist nach dieser Zeit homogen, und es wird direkt an einer 0,5 m langen beheizten, mit Multifill-Füllkörpern gefüllten Glaskolonne destilliert:

Einsatz: 2 907 g

| Fr. Nr. | Siede-bereich °C | Gewicht g | | Gewichts-prozent | Druck mbar | Verhältnis Rücklauf zur Abnahme |
|---|---|---|---|---|---|---|
| 1 | 93 bis 103 | a) | 359 | 12,4 | Normal-druck | 3:1 |
| | 42 bis 127 | b) | 1 283 | 44,6 | 133 | 10:1 |
| | | | 1 642 | 57,0 | | 30:1 |
| 2 | 127 bis 134 | | 36 | 1,3 | 133 | 30:1 |
| 3 | 134 bis 138 | | 1 094 | 38,0 | 133 | 30:1 |
| | | | | | | 5:1 |
| Rückstand | | | 102 | 3,5 | | |
| Kühlfalle | | | 5 | 0,2 | | |
| | | | 2 897 | 100,0 | | |

Die erste Fraktion trennt sich in zwei Phasen auf, in eine Ölphase (359 g) und eine Ameisensäurephase (1 283 g); letztere hat eine Säurezahl von 1 151,9 und besteht demnach zu 94,4% aus Ameisensäure. Die Ölphase hat nach GC-Analyse einen Gehalt an Cyclooctadien-1,5 von ca. 70% und an Umsetzungsprodukten (5 Komp.) von 1,5%. Ihr Ameisensäuregehalt liegt nach Säurezahl bei 4,2%.

Die Fraktion 2 ist ein Zwischenlauf und besteht zu 88,3% aus Produkten, die durch Addition von Ameisensäure an Cyclooctadien-1,5 entstanden sind.

Die dritte Fraktion besteht wie die 1 H- und 13 C-NMR-Spektren zeigen, zu 75% aus Cycloocten-4-ol-1-formiat. Der Gehalt an Bicyclooctanolen liegt bei 15 bis 22%.

Hieraus errechnet sich ein Umsatz an Cyclooctadien-1,5 von 80%. Die Ausbeute an Monoameisensäureester, bezogen auf umgesetzes Dien, liegt bei ca. 76% der Theorie.

Der Ameisensäureester wird in bekannter Weise in Gegenwart von Butyltitanat als Katalysator mit Isopropanol bei Temperaturen um 180 bis 190 °C umgeestert. Das anfallende Isopropylformiat destilliert man langsam, und nachdem kein Ameisensäureester mehr anfällt, destilliert man zunächst das überschüssige Isopropanol und direkt danach das Cycloocten-4-ol-1. Die Ausbeute bei der Herstellung des Alkohols aus dem Formiat liegt bei ca. 95% der Theorie.

Beispiele 2 und 3

Die Durchführung erfolgt wie im Beispiel 1 beschrieben. Die Reaktionszeit beträgt jeweils 10 Stunden, und die Temperaturen liegen zwischen 98 und 102 °C. Verändert wird lediglich das Molverhältnis der Einsatzstoffe. Die Ergebnisse gehen aus der folgenden Tabelle hervor:

| Bsp. | Molverhältnis COD[+]:Ams.[++] | Umsatz an COD | Ausbeute an Ester, bezogen auf umgesetztes COD | Gehalt an Cycl, Cycloocten-4-ol-1-formiat |
|------|------|------|------|------|
| 2 | 1:1,5 | 36,7% | 78,8% | 72% |
| 3 | 1:6 | 99,0% | 62,8% | 80% |

[+] Cyclooctadien-1,5
[++] Ameisensäure

Die Beispiele zeigen, dass das Molverhältnis der Ausgangsstoffe einen starken Einfluss auf den Umsatz ausübt.

Die Umesterung erfolgt wie im Beispiel 1 beschrieben.

Beispiele 4 und 5

Die Durchführung erfolgte nach den Angaben des Beispiels 1, verändert wurde jedoch die Temperatur. Im Beispiel 4 arbeitet man bei 60 °C, im Beispiel 5 bei 160 °C in einem Druckreaktor. Die Ergebnisse sind in der folgenden Tabelle zusammengestellt.

| Bsp. | Temp. °C | Versuchsdauer h | Umsatz an COD % | Ausbeute an Ester, bezogen auf umgesetztes COD % | Gehalt an Cycloocten-4-ol-1-formiat % |
|------|------|------|------|------|------|
| 4 | 60 | 20 | 43,7 | 77,9 | 78 |
| 5 | 160 | 3 | 51,6 | 53,23 | 72 |

Die Umsetzung erfolgt nach den Angaben des Beispiels 1.

## Patentansprüche

1. Verfahren zur Herstellung von Cycloocten-4-ol-1 mit einem Gehalt von über 70% durch Umsetzung von Cyclooctadien-1,5 mit Ameisensäure und anschliessende Umesterung des Cyclooctenylformiats mit einem niedrig siedenden Alkohol, dadurch gekennzeichnet, dass man Ameisensäure mit dem Cyclooctadien-1,5 ohne Katalysator bei 50 bis 200 °C, vorzugsweise bei 95 bis 105 °C, umsetzt, ohne weitere Aufarbeitungen das Reaktionsprodukt direkt destilliert und den erhaltenen Ameisensäureester in bekannter Weise mit einem niedrig siedenden Alkohol in dessen Formiat und zum Cycloocten-4-ol-1 umestert.

2. Verfahren nach Anspruch 1, dadurch gekennzeichnet, dass man ein Molverhältnis von Cyclooctadien-1,5 zur Ameisensäure von 1:0,1 bis 1:10, vorzugsweise von 1:1,5 bis 1:6, einsetzt.

## Revendications

1. Procédé de préparation de cyclo-octène-4-ol-1- d'une teneur supérieure à 70%, par réaction de cyclo-octadiène-1,5 sur de l'acide formique et trans-estérification subséquente du formiate de cyclo-octényle avec un acool à bas point d'ébullition, caractérisé par le fait que l'on fait réagir l'acide formique sur le cyclo-octadiène-1,5 sans catalyseur, à une température de 50 à 200 °C, de préférence à une température de 95 à 105 °C, que sans procéder à d'autres traitements, on distille directement le produit réactionnel et qu'on trans-estérifie l'ester formique obtenu, d'une manière connue en soi, avec un alcool à bas point d'ébullition pour obtenir le formiate correspondant et que l'on trans-estérifie en cyclo-octène-4-ol-1.

2. Procédé selon la revendication 1, caractérisé par le fait que l'on utilise un rapport molaire de 1:0,1 à 1:10, de préférence de 1:1,5 à 1:6, entre le cyclo-octadiène-1,5 et l'acide formique.

## Claims

1. A process for the production of cycloocten-4-ol-1 at a level in excess of 70% by reaction of cyclooctadiene-1.5 with formic acid and subsequent transestification of the resulting cyclooctenyl formate with a low boiling alcohol, characterised in that formic acid is reacted with the cyclooctadiene-1.5 at 50 to 200 °C, preferably 95 to 105 °C, in the absence of a catalyst, the reaction product is directly distilled without intervening working up and the formic acid ester is transesterified in a known manner with a low boiling alcohol to produce the formate thereof and cycloocten-4-ol-1.

2. A process according to claim 1, characterised in that a molar ratio of cyclooctadiene-1.5 to formic acid of from 1:0.1 to 1:10, preferably from 1:1.5 to 1:6, is employed.